(19) Europäisches Patentamt
European Patent Office
Office européen des brevets

(11) **EP 2 794 527 B1**

(12) **FASCICULE DE BREVET EUROPEEN**

(45) Date de publication et mention
de la délivrance du brevet:
**04.10.2017 Bulletin 2017/40**

(21) Numéro de dépôt: **12806596.8**

(22) Date de dépôt: **29.11.2012**

(51) Int Cl.:
**C07C 17/087** *(2006.01)*  **C07C 17/158** *(2006.01)*
**C07C 21/18** *(2006.01)*  **C07C 17/20** *(2006.01)*
**C07C 17/25** *(2006.01)*

(86) Numéro de dépôt international:
**PCT/FR2012/052752**

(87) Numéro de publication internationale:
**WO 2013/093272 (27.06.2013 Gazette 2013/26)**

(54) **PROCÉDÉ DE PRÉPARATION DE COMPOSES OLÉFINIQUES FLUORES**

VERFAHREN ZUR HERSTELLUNG FLUORIERTER OLEFINVERBINDUNGEN

METHOD FOR PREPARING FLUORINATED OLEFIN COMPOUNDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2011 FR 1162314**

(43) Date de publication de la demande:
**29.10.2014 Bulletin 2014/44**

(73) Titulaire: **ARKEMA FRANCE**
**92705 Colombes Cedex (FR)**

(72) Inventeur: **DUBOIS, Jean-Luc**
**F-69390 Millery (FR)**

(74) Mandataire: **Dang, Doris**
**ARKEMA FRANCE**
**Département Propriété Industrielle**
**420, rue d'Estienne d'Orves**
**92705 Colombes Cedex (FR)**

(56) Documents cités:
**WO-A1-2011/130108**

Remarques:
Le dossier contient des informations techniques présentées postérieurement au dépôt de la demande et ne figurant pas dans le présent fascicule.

**Description**

DOMAINE DE L'INVENTION

L'invention a pour objet un procédé de préparation de composés oléfiniques fluorés, notamment les (chloro)fluoropropènes et (chloro)fluorobutènes, et plus particulièrement, le composé fluoré 2,3,3,3-tétrafluoro-1-propène (HFO-1234yf).

ARRIERE-PLAN TECHNOLOGIQUE

Les hydrofluorocarbones (HFC) et en particulier les hydrofluorooléfines, telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

On connaît plusieurs procédés de fabrication du HFO-1234yf.

WO 2011/130108 A1 divulgue un procédé de fabrication du HFO-1234yf comprenant une étape pour préparer le 261bb à partir du 1250xf avec HF, une étape de chloration 261bb pour donner le 241bb, et une étape de réaction du 241bb avec HF en présence du dioxygène pour donner le 1234yf.

WO 2009/084703 décrit un procédé de fabrication du HFO-1234yf à partir de l'hexafluoropropène en passant par l'intermédiaire de l'hexafluoropropane, du pentafluoropropène, et du pentafluoropropane.

WO 2007/079431 décrit la préparation du HFO-1234yf par un procédé comprenant les étapes de fluoration de 2-chloro-3,3,3-trifluoro-1-propène (HCFO-1233xf) en 1,1,1,2-tétrafluoro-2-chloropropane (HFC-244bb), suivi d'une étape de déhydrochloration. Le produit HCFO-1233xf est préparé par fluoration du précurseur chloré correspondant (CCl$_2$=CClCH$_2$Cl).

WO 2008/054781 décrit une préparation du HFO-1234yf par réaction du 2,3-dichloro-1,1,1-trifluoropropane (HFC-243db) en présence de HF, sur un catalyseur notamment Cr/Co 98/2. Les produits de la réaction comprennent du HFO-1234yf et du 2-chloro-3,3,3-trifluoro-1-propène (HCFO-1233xf), ce dernier produit étant majoritaire; les autres produits 1-chloro-3,3,3-trifluoro-1-propène (HCFO-1233zd) ainsi que 1,1,1,2,2-pentafluoropropane (HFC-245cb) et 1,3,3,3-tétrafluoro-1-propène (HFO-1234ze) se forment aussi. Une température plus élevée favorise la production de l'isomère 1233zd. Le produit de départ 2,3-dichloro-1,1,1-trifluoropropane (HFC-243db) est indiqué comme étant obtenu par chloration du trifluoro-1-propène (TFP).

WO 2008/040969 et WO 2008/075017 décrivent une préparation sensiblement similaire. Il est indiqué que la réaction procède par déhydrochloration du HFC-243db en HCFO-1233 (tant xf que zd), suivi d'une réaction impliquant la formation de 1,1,1,2-tétrafluoro-2-chloropropane et la formation ultérieurement du 2,3,3,3-tétrafluoro-1-propène recherché par déhydrochloration. Le rapport HF:organiques est varié et il est indiqué que la réaction de déhydrochloration en HCFO-1233 (xf et zd) est favorisée par des ratios HF:organiques faibles tandis que la réaction de préparation du composé final recherché est favorisée par des ratios HF:organiques élevés. Le produit de départ 2,3-dichloro-1,1,1-trifluoropropane (HFC-243db) est indiqué comme étant obtenu par chloration du trifluoropropène ou du trifluoromethylpropène.

WO 2010/123154 décrit une préparation du HFO-1234yf par réaction du HCFO-1233xf avec de l'HF en présence d'oxygène et d'un catalyseur comprenant de l'oxyde de chrome CrO$_m$ avec $1,5 < m < 3$. Ce document enseigne l'utilisation d'un ratio molaire d'oxygène / HCFO-1233xf d'au plus égal à 1 pour améliorer la sélectivité.

Par ailleurs, le document US 2011/245548 décrit l'utilisation d'un ratio molaire d'oxygène par rapport à un composé chloré compris entre 0,1 et 1 pour augmenter la durée de vie d'un catalyseur dans la réaction de fluoration du pentachloropropane ou tetrachloropropène pour donner du HCFO-1233xf.

Les méthodes de préparation telles que précitées comprennent en général plusieurs étapes et nécessitent un investissement très coûteux. En outre, leur mise en oeuvre à l'échelle industrielle est souvent complexe. La présence des sous produits non valorisables ainsi que la durée de vie du catalyseur sont parmi les problèmes les plus fréquemment rencontrés lorsqu'on passe à l'échelle industrielle.

De plus , la présence d'oxygène en quantité telle que décrite dans l'art antérieur pose de nombreux problèmes : formation de sous produits oxygénés ; formation de l'eau qui en présence d'HF conduit à un milieu très corrosif et de sécurité (risque d'inflammabilité de l'effluent gazeux) dans le réacteur ou en aval du réacteur dans les unités de séparation.

Il existe un besoin d'un procédé de préparation de HFO-1234yf à partir d'un produit de départ qui soit aisément accessible, et facile à mettre en oeuvre conduisant « durablement » au produit recherché avec une sélectivité élevée, et avantageusement un rendement et/ou une conversion élevé.

**RESUME DE L'INVENTION**

L'invention fournit donc un procédé de préparation de 2,3,3,3-tétrafluoro-1-propène par fluoration en phase gazeuse avec de l'HF d'au moins un composé choisi parmi les halopropanes de formule $CX_3CHClCH_2X$, halopropènes de formules $CX_3CCl=CH_2$, $CClX_2CCl=CH_2$ et $CX_2=CClCH_2X$, avec X représentant indépendamment un atome de fluor ou de chlore, en présence du dioxygène et d'un catalyseur de fluoration maintenu en suspension dans un réacteur à lit fluidisé.

Selon un mode de réalisation, l'halopropane est choisi parmi le 2,3-dichloro-1,1,1-trifluoropropane et/ ou le 1,1,1,2,3-pentachloropropane (HFC-240db).

Selon un mode de réalisation, l'halopropène de formule $CX_3CCl=CH_2$ est choisi parmi le 2-chloro-3,3,3-trifluoro-1-propène.

Selon un mode de réalisation, le rapport HF/ composés à fluorer est surstoechiométrique.

Selon un mode de réalisation, les intermédiaires de réaction du 2,3,3,3-tétrafluoro-1-propène sont recyclés au réacteur.

**DESCRIPTION DETAILLEE DES MODES DE REALISATION**

L'invention utilise une étape de fluoration en phase gazeuse avec de l'HF et d'au moins un composé choisi parmi les halopropanes de formule $CX_3CHClCH_2X$, halopropènes de formules $CX_3CCl=CH_2$, $CClX_2CCl=CH_2$ et $CX_2=CClCH_2X$, avec X représentant indépendamment un atome de fluor ou de chlore, en présence du dioxygène et d'un catalyseur de fluoration maintenu en suspension dans un réacteur à lit fluidisé.

Le dioxygène peut être sous forme d'air, air enrichi ou oxygène de haute pureté.

Selon l'invention, cette étape est mise en oeuvre sous une pression supérieure à 1,5 bar absolu. Avantageusement, la pression est comprise entre 2 et 15 bar, notamment entre 2,2 et 5 bar.

Le ratio molaire HF:composés à fluorer est compris entre 50:1 et 1:1, de préférence 30:1 à 5:1, avantageusement entre 25:1 et 5:1.

La température de réaction peut être très variable. Par exemple, elle peut être comprise entre 200 et 500°C, de préférence entre 250°C et 400°C, avantageusement entre 275 et 380°C.

Le ratio molaire d'oxygène : composés à fluorer est compris entre 0,01 et 1, de préférence compris entre 0,05 et 0,2.

Le temps de contact (tc) entre le mélange gazeux comprenant les réactifs et le catalyseur, défini dans la présente invention suivant la formule ci-après :

$$tc \ (s.g/cm^3) \ = \ (W/F)\,x\,(273/(273+T))\,x\,((101325+P)/101325)$$

dans laquelle W représente la quantité de catalyseur dans le lit fluidisé en gramme, F représente la vitesse du flux gazeux dans les conditions normales de température et de pression, T la température de la réaction en °C et P la pression en Pascal et est compris entre 1 et 50 s et avantageusement compris entre 2 et 40 s.

La vitesse linéaire du flux gazeux comprenant les réactifs introduit dans le réacteur à lit fluidisé définie dans la présente invention par la formule ci-après :

$$Vl(cm/sec)= \ F \ x \ ((273+T)/273) \ x \ (101325/(101325+P)\,x \ S)$$

dans laquelle F, T et P sont définis comme ci-dessus et S est l'aire de la section interne du réacteur ($cm^2$) - aussi appelée vitesse linéaire en fût vide - et est compris entre 0,5 et 200 cm/s et avantageusement comprise entre 1 et 100 cm/s.

Le flux gazeux des réactifs peut être introduit dans le réacteur, en partie ou en totalité, par injection tangentielle de manière à créer un mouvement de turbulence contrôlé à travers le lit fluide, pour augmenter ainsi le temps de contact, améliorer l'homogénéité du catalyseur et/ou éviter l'agglomération des particules de catalyseurs.

Le produit de réaction peut être soumis à des étapes de séparation comme la distillation, le lavage, etc., de façon classique connue de l'homme du métier.

Selon l'invention, le flux sortant du réacteur fluidisé comprend du HFO-1234yf et au moins un composé choisi parmi le 1,1,1,2,2-pentafluoropropane, 2-chloro-3,3,3-trifluoro-1-propène et le 2-chloro,1,1,1,2 - tetrafluoropropane.

Ce flux peut en outre comprendre de l'HF et du HCl.

Le flux après séparation du HFO-1234yf et éventuellement HCl et ensuite recyclé au réacteur.

Selon un autre aspect, l'invention est un procédé de préparation du HFO-1234yf à partir du 1,1,1,2,3-pentachloropropane comprenant une étape de fluoration en phase gazeuse du 1,1,1,2,3-pentachloropropane avec de l'HF en présence du dioxygène et d'un catalyseur de fluoration maintenu en suspension dans un réacteur à lit fluidisé pour donner un flux comprenant du HFO-1234yf, 2-chloro-3,3,3-trifluoro-1-propène, 1,1,1,2,2-pentafluoropropane, de l'HF et HCl et une étape de séparation dans laquelle le HFO-1234yf et éventuellement HCl est séparé du flux avant d'être recyclé au réacteur lit fluidisé.

Le catalyseur mis en jeu est par exemple un catalyseur à base d'un métal notamment d'un métal de transition ou un dérivé oxyde ou halogénure ou oxyhalogénure d'un tel métal. Des catalyseurs sont par exemple $FeCl_3$, oxyfluorure de chrome, Ni (incluant les treillis de Ni mesh), $NiCl_2$, $CrF_3$, et leurs mélanges, par exemple Ni-Cr/$ALF_3$. D'autres catalyseurs possibles sont les catalyseurs supportés sur carbone, les catalyseurs à base d'antimoine, les catalyseurs à base d'aluminium (tel que $AlF_3$ et $Al_2O_3$ et oxyfluorure d'aluminium et alumine fluorée), de palladium, de platine, de rhodium et de ruthénium. On pourra se référer à la liste donnée dans le document US-P-5396000, colonne 1, ligne 50 à colonne 2, ligne 2 ou à la liste donnée dans WO2007/056194, page 16, lignes 13-23. On pourra aussi utiliser les catalyseurs décrits dans WO2008/040969, et notamment le Zn sur oxyde de chrome, traité par HF.

Les catalyseurs à base de chrome sont préférés.

Les catalyseurs à base de chrome peuvent comprendre au moins un co-catalyseur choisi (s) parmi le Co, Ti, V, Fe, Ge, As, Nb, Mo, Sb, W, Ta, P et Mn.

Sont également préférés des catalyseurs mixtes comprenant du chrome et au moins un métal choisi parmi le Ni, Mg et Zn.

Pour améliorer l'attrition de ces catalyseurs, on peut incorporer des composés tels que de la silice colloïdale, de l'alumine et/ou supporter la matière active sur ou dans des billes de carbure de silicium fluidisables.

Les catalyseurs sont mis en oeuvre avantageusement sous forme de poudre. Ils sont généralement obtenus par atomisation.

Le diamètre moyen des particules de catalyseurs est de préférence compris entre 20 et 200 microns, avantageusement compris entre 2 et 80 microns.

Les catalyseurs entraînés par le flux de réactifs, incluant les fines poudres produites par l'attrition, sont récupérés par des cyclones internes ou externes placés sur la ligne de traitement des effluents, ainsi que par des filtres de particules. Après élimination des fines, les catalyseurs récupérés dans le cyclone sont ensuite renvoyés dans le réacteur via une jambe d'élutriation.

Avantageusement, les catalyseurs sont soumis à un traitement d'activation en présence d'un courant d'agent oxydant tel que l'air, l'oxygène ou le chlore.

Avantageusement, les catalyseurs sont également soumis à une étape d'activation à l'aide d'un flux comprenant de l'acide fluorhydrique.

Selon un mode de réalisation, l'activation des catalyseurs peut être mise en oeuvre en deux étapes avec un traitement avec l'agent oxydant suivi de celui avec de l'HF.

Selon un autre mode de réalisation, l'activation des catalyseurs peut être mise en oeuvre en deux étapes avec un traitement avec de l'HF suivi de celui avec l'agent oxydant.

En fonction du catalyseur ou de la réaction, on peut effectuer plusieurs fois cette alternance (activations avec un traitement à l'air suivi de l'HF, de nouveau un traitement d'air suivi d'HF et ainsi de suite).

La température du traitement avec l'agent oxydant peut être comprise entre 250 et 500°C, de préférence entre 300 et 400°C pour une durée comprise entre 10 et 200 heures.

Celle du traitement avec l'HF peut être comprise entre 100 et 450°C, de préférence entre 200 et 300°C pour une durée comprise entre 1 et 50 heures.

Selon un autre mode de réalisation, l'activation des catalyseurs peut être mise en oeuvre en au moins une étape avec un traitement au mélange d'HF et d'agent oxydant. L'agent oxydant peut représenter entre 2 et 98 % molaire par rapport au mélange d'HF et agent oxydant et la température d'activation peut varier entre 200 et 450°C pour une durée comprise entre 10 et 200 heures.

L'activation de catalyseur peut être poursuivie par une réaction de fluoration en présence d'un agent oxydant, d'HF et d'au moins un composé choisi parmi un halopropane (s) de formules $CX_3CHClCH_2X$ et $CX_3CFXCH_3$, et/ou au moins un halopropène(s) de formules $CX_3CCl=CH_2$, $CClX_2CCl=CH_2$ et $CX_2=CClCH_2X$, avec X représentant indépendamment un atome de fluor ou de chlore. Le ratio molaire HF/halopropane et/ou halopropène peut être compris entre 2 et 40. Le ratio molaire agent oxydant/ halopropane et/ou halopropène peut être compris entre 0,04 et 2,5 La durée de cette étape d'activation par fluoration peut être comprise entre 6 et 100 heures et la température comprise entre 300 et 400°C.

A l'issue de cette étape d'activation, le catalyseur est de préférence soumis à un traitement à l'air avant de mettre en oeuvre le procédé de fabrication du 2,3,3,3-tetrafluoropropène.

Les étapes d'activation peuvent être mises en oeuvre à pression atmosphérique ou sous pression jusqu'à 20 bar.

L'activation des catalyseurs peut être mise en oeuvre dans le même réacteur que le procédé de fabrication selon la présente invention et présente l'avantage d'être plus pratique à mettre en oeuvre que dans un procédé en lit fixe.

Le procédé de la présente invention peut comprendre au moins une étape de régénération discontinue en présence du dioxygène. La température peut varier entre 250 et 500°C ; le temps de contact peut être compris entre 1 et 200 s et la durée de préférence comprise entre 10 et 200 h. La pression à laquelle la régénération catalytique peut être mise en oeuvre est de préférence comprise entre 1 et 20 bar abs.

Le réacteur en lit fluidisé est de préférence équipé d'échangeurs de chaleur plongeant dans le lit de solide, de manière à évacuer ou fournir les calories nécessaires à la réaction et/ou régénération, ainsi qu'aux phases transitoires.

**Revendications**

1.   Procédé de fabrication du 2,3,3,3-tétrafluoro-1-propène comprenant au moins une étape de fluoration en phase gazeuse avec de l'HF et d'au moins un composé choisi parmi les halopropanes de formule $CX_3CHClCH_2X$, halopro-pènes de formules $CX_3CCl=CH_2$, $CClX_2CCl=CH_2$ et $CX_2=CClCH_2X$, avec X représentant indépendamment un atome de fluor ou de chlore, en présence du dioxygène et d'un catalyseur de fluoration maintenu en suspension dans un réacteur à lit fluidisé ; l'étape de fluoration étant mise en oeuvre sous une pression supérieure à 1,5 bar absolu, le ratio molaire HF : composés à fluorer est compris entre 50 :1 et 1 :1, le ratio molaire oxygène :composés à fluorer est compris entre 0,01 et 1, le temps de contact entre le mélange gazeux comprenant les réactifs et le catalyseur, défini dans la présente invention suivant la formule ci-après :

```
tc (s.g/cm³) = (W/F)x(273/(273+T))x((101325+P)/101325)
```

dans laquelle W représente la quantité de catalyseur dans le lit fluidisé en gramme, F représente la vitesse du flux gazeux dans les conditions normales de température et de pression, T la température de la réaction en °C et P la pression en Pascal et est compris entre 1 et 50 s et la vitesse linéaire du flux gazeux comprenant les réactifs introduit dans le réacteur à lit fluidisé définie dans la présente invention par la formule ci-après :

```
Vl(cm/sec)= F x ((273+T)/273) x (101325/(101325+P)x S)
```

dans laquelle F, T et P sont définis comme ci-dessus et S est l'aire de la section interne du réacteur ($cm^2$) est compris entre 0,5 et 200 cm/s.

2.   Procédé selon la revendication 1 caractérisé en que le dioxygène est sous forme d'air, air enrichi ou oxygène de haute pureté.

3.   Procédé selon la revendication 1 ou 2 caractérisé en que l'halopropane est choisi parmi le 2,3-dichloro-1,1,1-trifluoropropane et/ ou le 1,1,1,2,3-pentachloropropane.

4.   Procédé selon la revendication 1 ou 2 caractérisé en que l'halopropène de formule $CX_3CCl=CH_2$ est choisi parmi le 2-chloro-3,3,3-trifluoro-1-propène.

5.   Procédé selon l'une quelconque des revendications précédentes caractérisé en que ratio molaire d'oxygène : composés à fluorer est compris entre 0,05 et 0,2.

6.   Procédé selon l'une quelconque des revendications précédentes caractérisé en que le temps de contact entre le mélange gazeux comprenant les réactifs et le catalyseur est compris entre 2 et 40s.

7.   Procédé selon l'une quelconque des revendications précédentes caractérisé en que le catalyseur est à base de chrome ou catalsyseur mixte comprenant le chrome éventuellement supporté.

8.   Procédé selon la revendication 7 **caractérisé en ce que** le diamètre moyen des particules de catalyseur est compris entre 20 et 200 microns.

9.   Procédé selon l'une quelconque des revendications précédentes caractérisé en que le catalyseur est soumis à une étape d'activation.

**Patentansprüche**

1. Verfahren zur Herstellung von 2,3,3,3-Tetrafluor-1-propen, das mindestens einen Schritt der Gasphasenfluorierung mindestens einer aus Halogenpropanen der Formel $CX_3CHClCH_2X$, Halogenpropenen der Formeln $CX_3CCl=CH_2$, $CClX_2CCl=CH_2$ und $CX_2=CClCH_2X$, wobei X unabhängig für ein Fluor- oder Chloratom steht, ausgewählten Verbindung mit HF in Gegenwart von Disauerstoff und einem in einem Wirbelschichtreaktor in Suspension gehaltenen Fluorierungskatalysator umfasst; wobei der Fluorierungsschritt unter einem Druck von mehr als 1,5 bar absolut durchgeführt wird, wobei das Molverhältnis HF:zu fluorierende Verbindungen zwischen 50:1 und 1:1 liegt, das Molverhältnis Sauerstoff:zu fluorierende Verbindungen zwischen 0,01 und 1 liegt, die Kontaktzeit zwischen der die Reaktanten enthaltenden Gasmischung und dem Katalysator, die bei der vorliegenden Erfindung gemäß nachstehender Formel definiert ist:

$$\mathtt{tc\ (s.g/cm^3)\ =}$$
$$\mathtt{(W/F)\,x\,(273/(273+T))\,x\,((101325+P)/101325)}$$

wobei W für die Katalysatormenge in der Wirbelschicht in Gramm steht, F für die Geschwindigkeit des Gasstroms unter normalen Temperatur- und Druckbedingungen steht, T für die Reaktionstemperatur in °C steht und P für den Druck in Pascal steht, zwischen 1 und 50 s liegt und die Lineargeschwindigkeit des die Reaktanten enthaltenden Gasstroms, der in den Wirbelschichtreaktor eingetragen wird, die bei der vorliegenden Erfindung durch nachstehende Formel definiert ist:

$$\mathtt{Vl\ (cm/s)\ =\ Fx((273+T)\,x\,(101325/(101325+P)\,xS)}$$

wobei F, T und P wie oben definiert sind und S für die innere Querschnittsfläche des Reaktors ($cm^2$) steht, zwischen 0,5 und 200 cm/s liegt.

2. Verfahren nach Anspruch 1, **dadurch gekennzeichnet, dass** der Disauerstoff in Form von Luft, angereicherter Luft oder hochreinem Sauerstoff vorliegt.

3. Verfahren nach Anspruch 1 oder 2, **dadurch gekennzeichnet, dass** das Halogenpropan aus 2,3-Dichlor-1,1,1-trifluorpropan und/oder 1,1,1,2,3-Pentachlorpropan ausgewählt wird.

4. Verfahren nach Anspruch 1 oder 2, **dadurch gekennzeichnet, dass** das Halogenpropen der Formel $CX_3CCl=CH_2$ aus 2-Chlor-3,3-trifluor-1-propen ausgewählt wird.

5. Verfahren nach einem der vorhergehenden Ansprüche, **dadurch gekennzeichnet, dass** das Molverhältnis Sauerstoff:zu florierende Verbindungen zwischen 0,05 und 0,2 liegt.

6. Verfahren nach einem der vorhergehenden Ansprüche, **dadurch gekennzeichnet, dass** die Kontaktzeit zwischen der die Reaktanten enthaltenden Gasmischung und dem Katalysator zwischen 2 und 40 s liegt.

7. Verfahren nach einem der vorhergehenden Ansprüche, **dadurch gekennzeichnet, dass** der Katalysator auf Chrom basiert oder ein chromhaltiger Mischkatalysator, der gegebenenfalls geträgert ist, ist.

8. Verfahren nach Anspruch 7, **dadurch gekennzeichnet, dass** der mittlere Durchmesser der Katalysatorteilchen zwischen 20 und 200 Mikron liegt.

9. Verfahren nach einem der vorhergehenden Ansprüche, **dadurch gekennzeichnet, dass** der Katalysator einer Aktivierungsstufe unterworfen wird.

**Claims**

1. Process for the manufacture of 2,3,3,3-tetrafluoro-1-propene comprising at least one stage of gasphase fluorination with HF of at least one compound chosen from halopropanes of formula $CX_3CHClCH_2X$, and halopropenes of

formulae $CX_3CCl=CH_2$, $CClX_2CCl=CH_2$ and $CX_2=CClCH_2X$, with X independently representing a fluorine or chlorine atom, in the presence of molecular oxygen and of a fluorination catalyst held in suspension in a fluidized bed reactor; the fluorination stage being carried out under a pressure of greater than 1.5 bar absolute, the HF:compounds to be fluorinated molar ratio is between 50:1 and 1:1, the oxygen: compounds to be fluorinated molar ratio is between 0.01 and 1, the contact time between the gas mixture comprising the reactants and the catalyst, defined in the present invention according to the formula below:

$$ct\ (s.g/cm^3)\ =\ (W/F)\,x\,(273/(273+T))\,x\,((101325+P)/101325)$$

in which W represents the amount of catalyst in the fluidized bed in grams, F represents the velocity of the gas flow under standard temperature and pressure conditions, T represents the reaction temperature in °C and P represents the pressure in pascals and is between 1 and 50 s and the linear velocity of the gas flow comprising the reactants which is introduced into the fluidized bed reactor, defined in the present invention by the formula below:

$$Lv(cm/sec)=\ F\ x\ ((273+T)/273)\ x\ (101325/(101325+P)\ x\ S)$$

in which F, T and P are defined as above and S is the area of the internal cross section of the reactor ($cm^2$), is between 0.5 and 200 cm/s.

2. Process according to Claim 1, **characterized in that** the molecular oxygen is in the form of air, enriched air or high-purity oxygen.

3. Process according to Claim 1 or 2, **characterized in that** the halopropane is chosen from 2,3-dichloro-1,1,1-trifluoropropane and/or 1,1,1,2,3-pentachloropropane.

4. Process according to Claim 1 or 2, **characterized in that** the halopropene of formula $CX_3CCl=CH_2$ is chosen from 2-chloro-3,3,3-trifluoro-1-propene.

5. Process according to any one of the preceding claims, **characterized in that** the oxygen:compounds to be fluorinated molar ratio is between 0.05 and 0.2.

6. Process according to any one of the preceding claims, **characterized in that** the contact time between the gas mixture comprising the reactants and the catalyst is between 2 and 40 s.

7. Process according to any one of the preceding claims, **characterized in that** the catalyst is based on chromium or mixed catalyst comprising chromium which is optionally supported.

8. Process according to Claim 7, **characterized in that** the mean diameter of the catalyst particles is between 20 and 200 microns.

9. Process according to any one of the preceding claims, **characterized in that** the catalyst is subjected to an activation stage.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2011130108 A1
- WO 2009084703 A
- WO 2007079431 A
- WO 2008054781 A
- WO 2008040969 A

- WO 2008075017 A
- WO 2010123154 A
- US 2011245548 A
- US 5396000 P
- WO 2007056194 A